# EUROPEAN PATENT SPECIFICATION

(11) **EP 1 176 143 B1**
(45) Date of publication and mention of the grant of the patent: **29.09.2004**
(21) Application number: 01306394.6
(22) Date of filing: 26.07.2001
(51) Int. Cl.: C07D 401/04

(54) **Anhydrous purification of nicotine using an ion exchange resin**
Wasserfreie Reinigung von Nikotin unter Benützung eines Cationenaustauscherharzes
La purification anhydre de nicotine au moyen d'une resine echangeuse d'ions

(30) Priority: 27.07.2000 US 221027 P
(43) Date of publication of application: 30.01.2002
(73) Proprietor: ROHM AND HAAS COMPANY, Philadelphia, Pennsylvania 19106-2399 (US)
(72) Inventor: Hughes, Lyn, Harleysville, Pennsylvania 19438 (US)
(74) Representative: Kent, Venetia Katherine

(56) References cited:
- CN-A- 1 136 563
- US-A- 3 390 685
- J.Walden, H.P.Gregor Princ. Appl. Water Chem. Proceedings of Rudolfs Research Conference, 4th (1967), Meeting date 1965, 491-501, discussion 501-4 XP001022663

## Description

### BACKGROUND OF THE INVENTION

The present invention relates to the anhydrous purification of nicotine using cation exchange resins.

Nicotine is a naturally occurring alkaloid that is found in the tobacco plant, *Nicotiana tobacum*. It finds great use in the pharmaceutical and agricultural industries. In the pharmaceutical industry it is extensively used in smoking cessation formulations. In this use the nicotine can be administered in the form of lozenges, chewing gum, and inhalers. Because these applications are for human ingestion it is required that the nicotine be of very high purity as defined in the US Pharmacopeia. In agriculture it is used as a pesticide and is usually formulated as the nicotine sulfate salt dissolved in water. The common concentration is 40% nicotine. When used as a pesticide it is not necessary to meet the same stringent purity requirements as for pharmaceutical use.

Typically, nicotine is produced by extraction from tobacco leaves or waste products from the manufacture of tobacco for smoking. This extraction has been achieved both by extraction with organic solvents and aqueous solvents. The extraction is followed by multiple purification steps. These steps can include liquid-liquid extraction, chromatography, distillation, and ion exchange absorption/elution. For the production of high purity nicotine the final steps include vacuum distillation. The primary purpose of the distillation is to separate the nicotine from colored impurities. It also serves to reduce the water content.

The quality of the nicotine will degrade if exposed to excessive heat, or to air. In both cases the nicotine will develop a yellow to brown coloration which is not acceptable when high purity nicotine is required. Nicotine is not a volatile compound (bp 247°C @ 745mm Hg) and very low pressures are typically used to prevent the use of excessive heat. The exposure to air can lead to shortened shelf life of high purity nicotine.

Ion exchange resins have been used for the purification of nicotine at different stages in its isolation. Said purification work was done in aqueous solutions. However, the use of aqueous solutions can lead to the need for removal of large quantities of water to make solutions of concentrations suitable for use in the industry, e.g., as a 40% solution of nicotine sulfate as an agricultural pesticide. The removal of the water can be a large energy cost and can produce hazardous waste. When ion exchange resins have been used in the purification of nicotine it is required that the nicotine be absorbed onto the resin. The nicotine is first extracted with the ion exchange resin and absorbed onto the resin and then it is eluted from said resin. See, Prabhu *et al*, Tobacco Research 18, 125-128, 1992; Narasimha *et al*, Research and Industry 37, 115-117, 1992; French patent #1473458; Badgett, Ind Eng Chem, 42(12) 1950, 2530-1; Bhat *et al*, Proc Nat Acad Sci India, 60(a), IV, 359-362, 1990; de Lucas, Ind Eng Chem Res, 37, 4783-4791, 1998.

Walden and Gregor (Principles and Applications of Water Chemistry, Proceedings of the Rudolfs Research Conference, 4^{th} 491-504, 1965), reported the results of using ion exchange resins with nicotine in non-aqueous systems. Their resins were strongly acidic cation exchange resins with a styrenic backbone and with pendant lauryl chains to increase lipophilicity. Use of an exotic eluant, 0.02N n-butylamine in n-heptane, enabled them to separate aniline and nicotine. Because of the use of n-butylamine, this approach is impractical for industrial scale purification. Also, CN1136563A, describes a multistep purification process whereby the aqueous extract is first treated with sulfuric acid to precipitate inorganic salts, then it is passed over a porous, strongly acidic cation exchange resin with a styrenic backbone, to absorb the nicotine. Elution is achieved with ammonium hydroxide mixed with sodium or potassium hydroxide. After concentrating the eluate by distillation it is then extracted with benzene. The final benzene solution is 40%. Removal of the benzene by distillation gives pure, colorless nicotine.

In the present invention, the impurities, e.g., the undesirable color-causing components, are preferentially held by the ion exchange resin, and clear, colorless nicotine is produced. The present invention is an improvement in the art because it does not involve distillation or the use of high temperature. It also has the potential for point-of-use application to avoid storage problems. Further, Applicants' preferred non aqueous solvents are essentially benign in humans. The most preferred solvent is approved by the FDA for use as the propellant in inhalers. Many are non-ozone depleting and non-flammable.

The following terms have the following meanings herein:

The term "water retention capacity," as used herein, is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity)

Further, ion exchange resins are characterized by their capacity to exchange ions. This is expressed as the "Ion Exchange Capacity." For cation exchange resins the term used is "Cation Exchange Capacity." The ion exchange capacity is measured as the number equivalents of an ion that can be exchanged and can be expressed with reference to the mass of the polymer (herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

Ion exchange resins are manufactured in different forms. These forms can include spherical and non-spherical particles with size in the range of 0.001mm to 2mm. The non-spherical particles are frequently manufactured by grinding of the spherical particles. Products made in this way typically have particle size in the range 0.001mm to 0.2mm. The spherical particles are frequently known in the art as 'Whole Bead.' The non-spherical particles are frequently known in the art as 'Powders.'

### STATEMENT OF THE INVENTION

In its various aspects the present invention provides a method for purifying nicotine and the use of a method comprising the steps of:
a. dissolving coloured nicotine in a non aqueous solvent to form a nicotine/non aqueous solvent solution;
b. passing said solution formed in step a. through a cation exchange resin to obtain a colorless solution,
c. evaporating said non aqueous solvent from said solution to obtain a colorless nicotine.

### DETAILED DESCRIPTION OF THE INVENTION

A method for purifying nicotine comprising the steps of:
a. dissolving coloured nicotine in a non aqueous solvent to form a nicotine/ non aqueous solvent solution;
b. passing said solution formed in step a. through a cation exchange resin to obtain a colorless solution,
c. evaporating said non aqueous solvent from said solution to obtain a colorless nicotine.

Specifically when using a non aqueous solvent, such as 1,1,1,2-tetrafluoroethane (TFE), the 'colored' impure nicotine, is charged to a suitable vessel, and then said vessel is evacuated to remove the air. TFE is then added, and the pressure is allowed to rise to the vapor pressure of the TFE (approximately 520 kPascals at room temperature) to maintain the TFE in the liquid state. The nicotine dissolves in the TFE, and then, while still under pressure, the nicotine and TFE are passed over a suitable ion exchange resin. The color is retained on the resin and the effluent nicotine solution is colorless. TFE is then removed from the solution by reducing the pressure slowly, and providing a heat source to maintain the temperature of the solution between room temperature and the boiling point of TFE. A temperature near room temperature is preferred to remove the TFE quickly. Because TFE has such a low boiling point it is expected to be removed essentially quantitatively at atmospheric pressure. The TFE can be recovered and reused by using a compressor and condenser, or a condenser at less than the boiling point of the TFE. The resulting nicotine is colorless and contains less water than the starting nicotine.

The ion exchange resin used in this invention can be regenerated for re-use using any of the regeneration methods known in the art, such as treatment with a strong acid.

Because of the simplicity of the invention it can be used as a point-of-use method for purifying colored nicotine, either because said nicotine was originally colored, or because it has developed color during storage.

The present invention is also useful when combined with a TFE based process for loading nicotine onto ion exchange resins such as taught in USSN , entitled: A Method for the Anhydrous Loading of Nicotine onto Ion Exchange Resins, filed concurrently with the present application, July , 2000. The TFE/nicotine solution from the ion exchange resin column can be used directly for the loading process without need to evaporate the TFE. This combination has the advantage that the colored nicotine can be used as the raw material for the process. The colored nicotine is significantly cheaper than high purity nicotine.

The present invention can also be used in combination with the extraction of nicotine from an aqueous extract of tobacco or tobacco products using TFE as taught in WO98/45013. The method of aqueous extraction is well known in the art. In this combination the TFE extract is passed immediately through the ion exchange resin without evaporating the TFE.

Ion Exchange resins useful in the practice of the present invention include, but are not limited to, styrenic strongly acidic cation exchange resins with sulfonic or phosphonic acid functionalities having a weight capacity of 0.1 to 8meq/g, styrenic weakly acidic cation exchange resins with carboxylic or phenolic acid functionalities having a weight capacity of 0.1 to 8.5meq/g, or acrylic or methacrylic weakly acidic cation exchange resins with a carboxylic acid functionality with weight capacity of 0.1 to 14meq/g, that are suitable for human and animal ingestion.

Preferred cationic exchange resins include, but are not limited to, styrenic weakly acidic cation exchange resin with a phenolic acid functionality with a weight capacity of 0.1 to 8.5meq/g or , a styrenic strongly acidic cation exchange resin with a sulfonic acid functionality with weight capacity of 0.1 to 8meq/g, and acrylic or methacrylic weakly acidic cation exchange resin with a carboxylic acid functionality with weight capacity of 0.1 to 14meq/g.

The more preferred cationic exchange resins include, but are not limited to, acrylic or methacrylic weakly acidic cation exchange resin with a carboxylic acid functionality with weight capacity of 0.1 to 14meq/g.

The most preferred cationic exchange resins are methacrylic weakly acidic cation exchange resin with a carboxylic acid functionality with weight capacity of 0.1 to 12meq/g.

Strongly acidic and weakly acidic cation exchange resins useful in the practice of the present invention are in the acid form or salt form or partial salt form.

Ion exchange resins useful in this invention are in powder or whole bead form.

The preferred ion exchange resins useful in this invention are in powder form.

The ion exchange resins useful in this invention have an amount of water between 0% and the water retention capacity of said resin.

The preferred ion exchange resins used in the invention have between 0% and 25% water.

The most preferred ion exchange resins used in the invention have between 0% and 10% water.

Nicotine useful in the practice of the present invention includes, but is not limited to that derived from the extraction of nicotine from the tobacco plant *Nicotiana tobacum*, and nicotine from any source that has developed color during storage.

The solvents that can be used in the invention are non aqueous solvents including, but not limited to, halogenated hydrocarbons, ketones, alcohols, ethers, hydrocarbons, esters, nitriles, and mixtures thereof.

The preferred non aqueous solvents useful in the present invention are fluorohydrocarbons with boiling points at atmospheric pressure between 30°C and -100°C.

The more preferred non aqueous solvents are:
trifluoromethane (CF₃H);
fluoromethane (CH₃F);
difluoromethane (CF₂H₂);
1,1-difluoroethane (CF₂HCH₃);
1,1,1-trifluoroethane (CF₃CH₃);
1,1,1,2-tetrafluroethane (CF₃CFH₂) (TFE) pentafluoroethane (CF₃CF₂H);
1,1,1,2,2-pentafluorpropane (CF₃CF₂CH₃);
1,1,1,2,2,3-hexafluoropropane (CF₃CF₂CFH₂);
1,1,1,2,3,3-hexafluoropropane (CF₃CFHCF₂H);.
1,1,1,3,3,3-hexafluropropane (CF₃CH₂CF₃);
1,1,2,2,3,3-hexafluoropropane (CF₂HCF₂CF₂H);
1,1,1,2,2,3,3-heptafluoropropane (CF₃CF₂CF₂);
1,1,1,2,3,3,3-heptafluoropropane (CF₃CFHCF₃);

The most preferred non aqueous solvent is 1,1,1,2-tetrafluoroethane (TFE) (CF₃CFH₂).

The preferred concentration of the nicotine to non aqueous solvent useful in the practice of the invention is from 0.01% to 20% by weight of nicotine.

The more preferred concentration of the nicotine to non aqueous solvent useful in the practice of the invention is from 0.1% to 10% by weight of nicotine.

The most preferred concentration of the nicotine to non aqueous solvent useful in the practice of the invention is from 0.1% to 2% by weight of nicotine.

The range of ratios of nicotine to ion exchange resin useful in the practice of this invention is 0.5:1 to 1000:1 by weight.

The preferred range of ratios of nicotine to ion exchange resin useful in the practice of this invention is 2:1 to 250:1 by weight.

The more preferred range of ratios of nicotine to ion exchange resin useful in the practice of this invention is 10:1 to 100:1 by weight.

The following non-limiting examples illustrate the practice of the present invention.

### EXAMPLE 1―NICOTINE PURIFICATION

Construct equipment comprising a 300ml stainless steel reservoir (the feed reservoir) connected to a 10mm diameter chromatography column, the effluent from which is fed to a second 300ml stainless steel reservoir (the receiver). Include valves and fittings in suitable places to allow complete evacuation of the system, charging of the non aqueous solution (TFE) to the feed reservoir, transfering the non aqueous solution from the feed reservoir, through the column and into the receiver. Charge 10g nicotine to the feed reservoir, and 10g of a methacrylic weakly acidic cation exchange resin with a carboxylic acid functionality with weight capacity of 10 to 11.1meq/g and in the powder form (such as Amberlite® IRP64 from Rohm and Haas Company) to the column. Evacuate the whole system to remove air. Close the system so that no air enters it. Charge 300g of TFE to the feed reservoir. The pressure of the system will rise to approximately 520 kPascals due to the vapor pressure of TFE. Agitate the feed reservoir for 5 minutes to dissolve the nicotine in the TFE. Now pass the solution through the ion exchange resin and into the receiver at a flow rate of approximately 25ml/min. The color-causing compounds are retained on the column, together with some of the nicotine. Immerse the receiver in a bowl of water at room temperature and then slowly open the receiver to the atmosphere to evaporate the TFE. The purified nicotine is left in the receiver.

### EXAMPLE 2―TFE RECOVERY

Proceed as in Example 1, except connect the receiver to a second receiver with a valve in between. When the nicotine solution has passed through the ion exchange resin, evacuate the second receiver and insert it in a bath of dry-ice and isopropanol (temperature approximately -68°C). Immerse the first receiver in a bath of water at room temperature and then slowly open the valve to the second receiver. The TFE will boil and the vapor will be condensed in the second receiver. The recovered TFE can be re-used.

### EXAMPLE 3―NICOTINE PURIFICATION

At a pressure in excess of 600 kPascals, feed nicotine and TFE into an in-line mixer at a ratio of 1:40 by weight. Feed this solution to a column (30cm long x 2.5cm diameter) of a methacrylic weakly acidic cation exchange resin with a carboxylic acid functionality with weight capacity of 10 to 11.1meq/g and in the powder form (such as Amberlite® IRP64 from Rohm and Haas Company) and collect the colorless effluent in a suitable pressure vessel that has been previously evacuated to remove any air, and operating at 350 kPascals, and heated to maintain a temperature of 15°C. The TFE will boil leaving the purified nicotine in the vessel. Pass the TFE vapor from the receiving vessel into a compressor to raise the pressure to >520 kPascals followed by a heat exchanger to cool the liquid TFE to 15-20°C. Continuously return this TFE to the in-line mixer. When all the nicotine has been purified, stop the TFE flow, and allow the pressure in the receiving vessel to drop to atmospheric pressure. The receiving vessel contains the purified nicotine.

### EXAMPLE 4―NICOTINE PURIFICATION/LOADING

Using equipment similar to that used in Example 3, produce 18kg of purified nicotine, but operate the receiving vessel at 500-600 kPascals so that the TFE does not boil. The compressor is not needed at this stage. When the purification is complete, transfer the solution to another evacuated vessel that contains 100kg of a methacrylic weakly acidic cation exchange resin with a carboxylic acid functionality with weight capacity of 10 to 11.1meq/g and in the powder form (such as Amberlite® IRP64 from Rohm and Haas Company). Mix the slurry for at least 8 hours to allow the nicotine to be absorbed by the resin. Using the compressor as in Example 3, evaporate and recover the TFE. Remove resin loaded with nicotine (118kg) from the vessel.

## Claims

1. A method for purifying nicotine comprising the steps of
a. dissolving colored, nicotine in a non aqueous solvent to form a nicotine /non aqueous solvent solution;
b. passing said solution formed in step a. through a cation exchange resin to obtain a colorless solution,
c. evaporating said non aqueous solvent from said solution to obtain a colorless nicotine.

2. A method according to Claim 1 wherein said non aqueous solvent is a fluorohydrocarbon with a boiling point at atmospheric pressure between 30°C and -100°C.

3. A method according to Claim 2 wherein said non aqueous solvent is 1,1,1,2-tetrafluorethane.

4. A method according to any one of the preceding claims, wherein the concentration of the nicotine to solvent is 0.01% to 20% by weight of nicotine.

5. A method according to any one of the preceding claims, wherein the ratio of nicotine to ion exchange resin is 0.5:1 to 1000:1 by weight.

6. A method according to any one of the preceding claims, wherein said cation exchange resin is a weakly acidic resin with an acrylic or methacrylic backbone and a carboxylic acid functionality.

7. A method according to Claim 6, wherein said cation exchange resin is a weakly acidic resin with a methacrylic backbone and a carboxylic acid functionality.

8. A method according to any one of the preceding claims, wherein said cation exchange resin is in whole bead form.

9. A method according to any one of claims 1 to 7, wherein said cation exchange resin is in powder form.

10. A method according to any one of the preceding claims wherein said cation exchange resin contains an amount of water between 0% and the water retention capacity of said resin.

11. Use of a method comprising the steps of:
a. dissolving coloured nicotine in a non-aqueous solvent to form a nicotine/non-aqueous solvent solution;
b. passing said solution formed in step through a cation exchange resin to obtain a colourless solution; and
c. evaporating said non-aqueous solvent from said solution formed in step b.; to produce colourless nicotine.

## Patentansprüche

1. Verfahren zur Reinigung von Nikotin, umfassend die Schritte:
a. Lösen von gefärbtem Nikotin in einem nicht wässrigen Lösemittel, um eine Nikotin/nicht wässrige Lösemittel-Lösung zu bilden;
b. Führen der in Schritt a. gebildeten Lösung durch ein Kationenaustauschharz, um eine farblose Lösung zu erhalten;
c. Verdampfen des nicht wässrigen Lösemittels aus der Lösung, um ein farbloses Nikotin zu erhalten.

2. Verfahren nach Anspruch 1, worin das nicht wässrige Lösemittel ein Fluorkohlenwasserstoff mit einem Siedepunkt bei atmosphärischem Druck zwischen 30°C und -100°C ist.

3. Verfahren nach Anspruch 2, worin das nicht wässrige Lösemittel 1,1,1,2-Tetrafluorethan ist.

4. Verfahren nach einem der vorangehenden Ansprüche, worin die Konzentration von Nikotin zu Lösemittel 0,01 Gew.-% bis 20 Gew.-% von Nikotin beträgt.

5. Verfahren nach einem der vorangehenden Ansprüche, worin das Verhältnis von Nikotin zu Ionenaustauschharz 0,5:1 bis 1000:1, bezogen auf das Gewicht, beträgt.

6. Verfahren nach einem der vorangehenden Ansprüche, worin das Kationenaustauschharz ein schwach saures Harz mit einem Acryl- oder Methacryl-Backbone und einer Carbonsäurefunktionalität ist.

7. Verfahren nach Anspruch 6, worin das Kationenaustauschharz ein schwach saures Harz mit einem Methacryl-Backbone und einer Carbonsäurefunktionalität ist.

8. Verfahren nach einem der vorangehenden Ansprüche, worin das Kationenaustauschharz in der Form ganzer Beads vorliegt.

9. Verfahren nach einem der Ansprüche 1 bis 7, worin das Kationenaustauschharz in Pulverform vorliegt.

10. Verfahren nach einem der vorangehenden Ansprüche, worin das Kationenaustauschharz eine Menge an Wasser zwischen 0% und der Wasserrückhaltekapazität des Harzes enthält.

11. Verwendung eines Verfahrens umfassend die Schritte:
a. Lösen von gefärbtem Nikotin in einem nicht wässrigen Lösemittel, um ein Nikotin/nicht wässriges Lösemittel-Lösung zu bilden;
b. Führen der in Schritt a. gebildeten Lösung durch ein Kationenaustauschharz, um eine farblose Lösung zu erhalten; und
c. Verdampfen des nicht wässrigen Lösemittels von der in Schritt b. gebildeten Lösung;
um farbloses Nikotin herzustellen.

## Revendications

1. Procédé de purification de nicotine, comprenant les étapes suivantes :
a. dissoudre de la nicotine colorée dans un solvant non aqueux, pour former une solution nicotine/solvant non aqueux ;
b. passer ladite solution formée à l'étape a. à travers une résine échangeuse de cations, pour obtenir une solution incolore ;
c. évaporer ledit solvant non aqueux de ladite solution, pour obtenir une nicotine incolore.

2. Procédé selon la revendication 1, dans lequel ledit solvant non aqueux est un hydrocarbure fluoré possédant un point d'ébullition à la pression atmosphérique compris entre 30 °C et -100 °C.

3. Procédé selon la revendication 2, dans lequel ledit solvant non aqueux est du 1,1,1,2-tétrafluoroéthane.

4. Procédé selon l'une quelconque des revendications précédentes, dans lequel la concentration de la nicotine dans le solvant est comprise entre 0,01 % et 20 % en poids de nicotine.

5. Procédé selon l'une quelconque des revendications précédentes, dans lequel le rapport entre nicotine et résine échangeuse d'ions est compris entre 0,5:1 et 1 000:1 en poids.

6. Procédé selon l'une quelconque des revendications précédentes, dans lequel ladite résine échangeuse de cations est une résine faiblement acide ayant un squelette acrylique ou méthacrylique et une fonctionnalité acide carboxylique.

7. Procédé selon la revendication 6, dans lequel ladite résine échangeuse de cations est une résine faiblement acide ayant un squelette méthacrylique et une fonctionnalité acide carboxylique.

8. Procédé selon l'une quelconque des revendications précédentes, dans lequel ladite résine échangeuse de cations se présente sous une forme de perles entières.

9. Procédé selon l'une quelconque des revendications 1 à 7, dans lequel ladite résine échangeuse de cations se présente sous la forme d'une poudre.

10. Procédé selon l'une quelconque des revendications précédentes, dans lequel ladite résine échangeuse de cations contient une quantité d'eau comprise entre 0 % et la capacité de rétention d'eau de ladite résine.

11. Utilisation d'un procédé comprenant les étapes suivantes :
a. dissoudre de la nicotine colorée dans un solvant non aqueux, pour former une solution nicotine/solvant non aqueux ;
b. passer ladite solution formée à l'étape a. à travers une résine échangeuse de cations, pour obtenir une solution incolore ; et
c. évaporer ledit solvant non aqueux de ladite solution formée à l'étape b, pour obtenir une nicotine incolore.
